(19) Europäisches Patentamt
European Patent Office
Office européen des brevets

(11) **EP 2 276 721 B1**

(12) **EUROPÄISCHE PATENTSCHRIFT**

(45) Veröffentlichungstag und Bekanntmachung des Hinweises auf die Patenterteilung:
**29.08.2018   Patentblatt 2018/35**

(21) Anmeldenummer: **09737927.5**

(22) Anmeldetag: **10.03.2009**

(51) Int Cl.:
*C07C 209/78* (2006.01)    *C07C 211/50* (2006.01)

(86) Internationale Anmeldenummer:
**PCT/EP2009/052768**

(87) Internationale Veröffentlichungsnummer:
**WO 2009/132883 (05.11.2009 Gazette 2009/45)**

(54) **VERFAHREN ZUR HERSTELLUNG VON DIAMINODIPHENYLALKANEN**

METHOD FOR PRODUCING DIAMINODIPHENYL ALKANES

PROCEDE DE PRODUCTION DE DIAMINODIPHENYLALCANES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **30.04.2008   DE 102008001469**

(43) Veröffentlichungstag der Anmeldung:
**26.01.2011   Patentblatt 2011/04**

(73) Patentinhaber: **Evonik Degussa GmbH**
**45128 Essen (DE)**

(72) Erfinder:
• **KRECZINSKI, Manfred**
**44652 Herne (DE)**
• **GRUND, Gerda**
**48653 Coesfeld (DE)**

(56) Entgegenhaltungen:
**EP-A1- 0 043 933     WO-A2-2009/132906**
**US-A- 4 554 378**

• **SIRIL ET AL: "New polystyrene sulfonic acid resin catalysts with enhanced acidic and catalytic properties" JOURNAL OF MOLECULAR CATALYSIS. A, CHEMICAL, ELSEVIER, AMSTERDAM, NL, Bd. 279, Nr. 1, 5. Dezember 2007 (2007-12-05), Seiten 63-68, XP022376501 ISSN: 1381-1169**

**Beschreibung**

Die Erfindung betrifft ein Verfahren zur Herstellung von Diaminodiphenylalkanen, insbesondere von Diaminodiphenylmethan.

Es ist bekannt, Diaminodiphenylalkane durch Kondensation von einem aromatischen Amin wie Anilin und Aldehyd an sauren Katalysatoren herzustellen.

Die Reaktion erfolgt so, dass zunächst aus einem aromatischen Amin (Anilin) und Aldehyd N-Alkyl-Verbindungen gebildet werden. Diese Vorkondensate reagieren dann in Gegenwart von sauren Katalysatoren weiter zu Aminalen. Diese Aminale lagern sich anschließend unter Einwirkung eines sauren Katalysators zu Diaminodiphenylalkanen um.

Bei der Reaktion entstehen verschiedene Isomere der Diaminodiphenylalkane. Es entstehen Mischungen aus 2,2'-, 2,4'- und 4,4'-Diaminodiphenylalkanen. Weiterhin entstehen als Nebenprodukte höhere Kondensationsprodukte mit drei bis sechs Kernen sowie N-Alkyl-Verbindungen.

Nach dem bekannten Stand der Technik werden die erhaltenen Diaminodiphenylalkane weiter verarbeitet zu Diisocyanaten wie beispielsweise Diisocyanatodicyclohexylmethan oder Diisocyanatodiphenylmethan bzw. anderen Diisocyanaten. Diese Verbindungen sind wichtige Lackrohstoffe und Rohprodukte, beispielsweise für die Herstellung von Polyurethanen.

Im Stand der Technik wurde häufig die Herstellung von Diaminodiphenylalkanen aus der Kondensation von Anilin und Aldehyd, insbesondere Anilin und Formaldehyd, durchgeführt. Dabei wurde je nach Reaktionsvariante entweder zunächst das Kondensationsprodukt aus Anilin und Formaldehyd hergestellt und dieses dann in Gegenwart von Säuren wie beispielsweise Salzsäure umgelagert oder aber die Kondensation wurde bereits in Gegenwart von Säuren unter Umlagerungsbedingungen durchgeführt.

Ein Nachteil dieses Vorgehens ist es, dass bei der homogenen Katalyse mit Mineralsäuren salzhaltige Abwässer anfallen, die bei der Neutralisation der Säuren entstehen. Darüber hinaus führen die wässrigen Mineralsäuren zu Korrosionsproblemen in den Herstellungsanlagen. Deshalb wurden im weiteren Stand der Technik Verfahren entwickelt, bei denen entsprechende heterogene Katalysatoren eingesetzt werden. Dabei werden neben sauren Ionenaustauschern auch saure synthetische oder natürliche Silicium- oder Aluminiumoxide eingesetzt wie Zeolite oder Tonmineralien.

In der US 4,294,981 wird in einem derartigen Verfahren die Kondensation in Gegenwart einer starken wässrigen Säure durchgeführt, wonach durch Lösungsmittelextraktion die Säure entfernt wird. Die Umlagerung wird wiederum in Gegenwart von starker Säure, die in geringerer Menge eingesetzt wird, durchgeführt. Diatomeenerde, Tone oder Zeolite können als Katalysator in dieser Reaktionsstufe eingesetzt werden.

Die DE-A-12 30 033 beschreibt ein Verfahren zur Herstellung von Diaminodiphenylalkanen. Dabei wird siliciumhaltiger Ton, ein synthetischer Siliciumdioxid-Aluminiumoxid-Katalysator oder ein Magnesiumoxid-Aluminiumoxid-Katalysator eingesetzt.

Ein weiteres Reaktionsverfahren zur Herstellung von Diaminodiphenylalkanen ist in der DE-A-14 93 431 beschrieben. Dort wird Siliciumdioxid, Siliciumdioxid-Aluminiumoxid oder säurebehandeltes Aluminiumoxid als Katalysator eingesetzt. Bevorzugt sind Kieselgel oder bentonitartiger Ton, der Siliciumdioxid und Aluminiumoxid enthält und vorzugsweise säureaktiviert ist.

Die US 4,071,558 beschreibt ein Herstellungsverfahren zur Herstellung von Diaminodiphenylalkanen, bei dem ein mit Säure aktivierter Tonkatalysator, ein Siliciumdioxid-Aluminiumoxid haltiger Crack-Katalysator oder ein Siliciumdioxid-Magnesiumoxid-Katalysator eingesetzt wird.

Die US 4,039,580 beschreibt ein Herstellungsverfahren, bei dem die Kondensation von Anilin und Formaldehyd in Abwesenheit eines Katalysators ausgeführt wird und das Kondensationsprodukt dann in Gegenwart von Diatomeenerde, Tone oder Zeoliten weiter umgesetzt wird zum Diaminodiphenylmethan. Ähnliche Umsetzungen beschreibt auch die US 4,039,581.

Die Katalysatoren aus der Gruppe Magnesium- oder Aluminiumoxide, Tonkatalysatoren oder Siliciumdioxid-Katalysatoren haben sich aufgrund ihrer hohen Preise, der geringen Aktivitäten, der nicht gleichbleibenden Qualität und mangelhafter Katalysatorstandzeiten nicht bewährt.

Im neueren Stand der Technik wird daher zur Herstellung von Diaminodiphenylalkanen als Katalysator ein Ionenaustauscher vorgeschlagen, der saure Gruppen besitzt. So beschreibt die EP 0 043 933 A1 ein Verfahren zur Herstellung von Polyamingemischen mit einem hohen Anteil an 4,4'-Diaminodiphenylmethan und einem niedrigen Anteil an 2,4'-Diaminodiphenylmethan, bei dem als Katalysator ein Ionenaustauscher auf der Basis eines Divinylbenzol/Styrol-Copolymerisats eingesetzt wird. Dieser Ionenaustauscher besitzt Sulfonsäuregruppen, eine spezifische Oberfläche von 2 bis 40 m$^2$/g und eine Porenweite von 0,5 bis 40 nm. Als saure Gruppen für den Katalysator werden Sulfonsäuregruppen verwendet. Die Ausbeuten bei diesem Verfahren liegen im Bereich von 60 bis 78 %. Mit dem sulfonierten Styrol-Divinylbenzol-copolymer-Katalysator lassen sich Diaminodiphenylmethane herstellen, die einen großen Gehalt an 4,4'-Diaminodiphenylmethan besitzen. Dieses Isomer wird insbesondere benötigt zur weiteren Verarbeitung, nämlich zur Umsetzung in entsprechende Diisocyanate der Diphenylmethan-Reihe, die die Ausgangsmaterialien bei der Herstellung von Polyurethanen darstellen oder als Lackrohstoffe verwendet werden. Die Druckschrift beschreibt weiterhin, dass der Anteil an 2,2'- und 2,4'-Diisocyanatodiphenylmethan-Verbindungen möglichst niedrig sein muss, weil diese Isomere für viele Anwendungsgebiete im Polyisocyanat-Bereich nicht erwünscht sind. Gemäß dem Stand der Technik der EP 0 043 933 werden die erhaltenen Diaminodiphenylmethan-Verbindungen unmittelbar einer Phosgenierung unterzogen, um entsprechende Diisocyanate herzustellen.

Das in der EP 0 043 933 beschriebene Verfahren zur Herstellung von Diaminodiphenylalkanen hat den Nachteil, dass es geringe Ausbeuten besitzt und trotz hoher Reaktionstemperatur sehr lange Reaktionszeiten notwendig sind, um eine technisch vertretbare Ausbeute zu erzielen. Ein weiterer Nachteil ist es, dass bei dem Verfahren des Standes der Technik ein nur geringer Anteil an 2,4'-Isomer entsteht.

Neben aromatischen Isocyanaten sind in einigen speziellen Bereichen die entsprechenden aliphatischen Isocyanate von besonderer Bedeutung.

Die nächste Stufe im Herstellungsprozeß von aliphatischen Isocyanaten, ist die Hydrierung des aromatischen Ringes der Diaminodiphenylalkane.

Bei der Hydrierung des Diaminodiphenylmethans entsteht aus dem 4,4'-Isomer das 4,4'-trans/trans-, cis/cis- und cis/trans-Diaminodicyclohexylmethan (PACM). Der Gehalt an trans/trans-4,4'-Diaminodicyclohexylmethan hat einen erheblichen Einfluss auf die Kristallisationsneigung des Diisocyanats. Wenn bei dem, aus PACM durch Phosgenierung oder andere Verfahren hergestelltem Diisocyanat, der trans/trans-4,4'-Anteil des Produktes zu hoch ist, kann das Diisocyanatodicyclohexylmethan bereits bei Raumtemperatur Kristalle bilden, was für die Weiterverarbeitung zu Polyurethanen hinderlich ist. Vor der Weiterverarbeitung müssen daher aufwendige Verfahrensschritte vorgenommen werden, um den Gehalt an 4,4'-Isomer auf einen akzeptablen Wert zu reduzieren, so dass eine Kristallitbildung nicht mehr auftritt. Dies erfolgt üblicherweise durch Anreicherung des 2,4'-Isomers.

Eine weitere Anforderung an den Isomerengehalt bei der Herstellung des Diaminodiphenylmethans ist es, dass ein möglichst geringer Anteil an 2,2'-Isomer vorhanden sein muss, da dieses Isomer im späteren Verarbeitungsschritt zu Polyurethanen einen Kettenabbruch bei der Polymerisationsreaktion verursacht.

Um diesen Zusatzaufwand zu vermeiden ist es für diesen Reaktionsweg wichtig, dass bereits bei der Herstellung von Diaminodiphenylmethan ein bestimmtes Isomerenverhältnis erzielt wird.

Nach dem bisherigen Stand der Technik wird dieses Isomerenverhältnis dadurch erhalten, dass das Diaminodiphenylmethan in aufwändiger Weise aufgereinigt und destilliert wird, um die Isomeren in dem für die Weiterverarbeitung notwendigen Verhältnis bereitstellen zu können.

Die technische Aufgabe der Erfindung ist es daher, ein Verfahren zur Herstellung von Diaminodiphenylalkanen zur Verfügung zu stellen, das direkt zu dem spezifischen Isomerenverhältnis führt, das man für die Weiterverarbeitung zum aliphatischen Diisocyanat und der entsprechenden Umsetzung zu Polyurethanen benötigt. Eine weitere technische Aufgabe der Erfindung ist es, ein Verfahren zu entwickeln das wirtschaftlicher arbeitet, weil es geringere Reaktionsdauer benötigt und zu den gewünschten Ausbeuten der Isomere führt. Gleichzeitig soll auch das Entstehen von Abwasser mit Salzfracht vermieden werden.

Diese technische Aufgabe wird gelöst durch ein Verfahren zur Herstellung von Diaminodiphenylalkanen wobei ein aromatisches Amin, das substituiert oder unsubstituiert sein kann, mit einem $C_1$-$C_3$ Aldehyd in Gegenwart eines heterogenen Katalysators umgesetzt wird, wobei der Katalysator ein mesoporöser saurer Ionenaustauscher auf Basis eines Divinylbenzol/Styrol-Copolymerisates ist und der Katalysator acide Zentren gemessen nach DIN 54 403 in einer Konzentration von 2 bis 6 eq/kg aufweist und der durchschnittliche Porendurchmesser der Katalysatorteilchen gemessen nach ASTM D 4222 22 bis 30 nm beträgt, und wobei der Gehalt an Mehrkernverbindungen im gebildeten Reaktionsgemisch > 10 und ≤ 15 Gew.-% und wobei die Reaktionstemperatur der katalytischen Reaktion im Bereich von 80 bis 140°C liegt, mit der Maßgabe , dass der Katalysator ausgewählt wird aus der Gruppe bestehend aus Amberlyst 35Wet, Amberlyst 39Wet und Amberlyst 70Wet. Unter mesoporösen Ionenaustauschern werden solche verstanden, die einen mittleren Porendurchmesser gemessen nach ASTM D 4222 von 2 bis 50 nm besitzen. Erfindungsgemäß werden jedoch mesoporöse Ionenaustauscher mit einem durchschnittlichen Porendurchmesser der Katalysatorteilchen gemessen nach ASTDM D 4222 von 22 bis einschließlich 30 nm eingesetzt.

Als Aldehyd wird vorzugsweise Formaldehyd eingesetzt. Formaldehyd kann als wässrige Formalinlösung oder auch gasförmig als Formaldehyd eingesetzt werden. Weiterhin können auch Stoffe verwendet werden, die unter den Reaktionsbedingungen Formaldehyd abspalten, wie beispielsweise Trioxymethylen oder Paraformaldehyd. In bevorzugter Weise wird für die Reaktion eine wässrige Formalinlösung eingesetzt.

Als aromatische Amine können substituierte oder unsubstituierte Amine eingesetzt werden. In bevorzugter Weise sollten die Amine, falls sie substituiert sind, keinen Substituenten in Parastellung besitzen. Geeignete aromatische Amine sind beispielsweise N-Methylanilin, N-Ethylanilin, o-Toluidin, o-Chloranilin, m-Chloranilin, o-Anisidin, 2,3-Xylidin, 3,5-Xylidin, o-Cyclohexylanilin, o-Benzylanilin, alpha-Naphtylanilin, Methylmercaptoanilin oder Anilin. Besonders bevorzugt als aromatisches Amin ist die Verwendung von Anilin.

Bei dem erfindungsgemäßen Herstellungsverfahren werden folgende Zusammensetzung und Isomerenverhältnisse erhalten, die vorzugsweise wie folgt verteilt sind:

64 bis 85 Gew.-% 4,4'-Diaminodiphenylalkan,

3 bis 20 Gew.-%, vorzugsweise 7 bis 17 Gew.-% 2,4'-Diaminodiphenylalkan und

$\geq$ 1 Gew.-% bis $\leq$ 2 Gew.-%, 2,2'-Diaminodiphenylalkan.

Mit dem erfindungsgemäßen Verfahren wird vorzugsweise Diaminodiphenylmethan der nachfolgenden Isomerenzusammensetzung hergestellt:

64 bis 85 Gew.-% 4,4'-Diaminodiphenylmethan,

3 bis 20 Gew.-%, vorzugsweise 7 bis 17 Gew.-%, 2,4'-Diaminodiphenylmethan und

$\geq$ 1 Gew.-% bis $\leq$ 2 Gew.-%, 2,2'-Diaminodiphenylmethan.

Der Anteil an Mehrkernverbindungen im Isomerengemisch des Diaminodiphenylalkans, insbesondere des Diaminodiphenylmethans, beträgt > 10 Gew.-% bis $\leq$ 15 Gew.-%, in bevorzugter Weise < 15 Gew.-%. Unter Mehrkernverbindungen versteht man im Rahmen der Erfindung solche Moleküle mit mehr als zwei aromatischen Kernen, insbesondere Phenylringe.

Die Verunreinigungen durch N-Methylverbindungen im Isomerengemisch des Diaminodiphenylalkans, insbesondere Diaminodiphenylmethan, betragen $\leq$ 1,0 Gew.-%, vorzugsweise $\leq$ 0,5 Gew.-% und besonders bevorzugt $\leq$ 0,3 Gew.-%.

Diese Isomerenverteilung ist besonders geeignet, um über Diaminodicyclohexylmethan zu der entsprechenden Diisocyanatodicyclohexylmethan-Verbindung weiterverarbeitet zu werden. Das Isomerenverhältnis, das beim erfindungsgemäßen Verfahren erhalten wird, bestimmt auch das anschließende Isomerenverhältnis in der Diisocyanat-Verbindung.

Mit dem erfindungsgemäßen Verfahren wird bereits bei der Diaminodiphenylalkan-Herstellung das geforderte Isomerenverhältnis erreicht, ohne dass durch zusätzliche Trennungsverfahren, die aufwändig und teuer sind, dieses Isomerenverhältnis erst eingestellt werden muss. Auch sind diese Aufreinigungsverfahren deshalb nachteilig weil es sich hier um sehr reaktive Substanzen handelt, sodass auch bei Aufreinigung oder Destillation mögliche Nebenreaktionen auftreten können.

Ein weiterer Vorteil des erfindungsgemäßen Verfahrens ist es, dass der Anteil an unerwünschten Nebenprodukten, insbesondere N-Methylverbindungen, sehr gering ist. N-Methylverbindungen führen bei der Weiterverarbeitung zum Diisocyanat zu unerwünschten Monoisocyanaten und damit zur Qualitätverschlechterung des Produktes.

Die Verfahren des Standes der Technik, die mit sauren Ionenaustauschern als Katalysatoren arbeiten, führen zu deutlich höheren Isomerenanteilen von 4,4'-Diaminodiphenylalkan. So liegen die entsprechenden Gehalte in den Beispielen der Druckschrift EP 0 043 933 bei 94 Gew.-%, 92 Gew.-% und 91 Gew.-%. Diese Isomerengehalte sind jedoch zu hoch, da bei der Weiterverarbeitung zur aliphatischen Diisocyanat-Verbindung eine Kristallisation eintritt, die unerwünscht ist.

Die gemäß EP 0 043 933 erhaltenen Diaminodiphenylalkane müssen daher durch zusätzliche Verfahrensschritte, wie Destillation, auf das gewünschte Isomerenverhältnis von 64 bis 85 Gew.-% 4,4'-Diaminodiphenylalkan, 3 bis 20 Gew.-% 2,4'-Diamiodiphenylalkan und $\geq$ 1,0 Gew.-% bis $\leq$ 2 Gew.-% 2,2'-Diaminodiphenylalkan eingestellt werden.

Ein weiterer Vorteil des erfindungsgemäßen Verfahrens liegt in seiner ökologischen und ökonomischen Ausführung. In bevorzugter Weise wird die katalytische Reaktion bei einer Reaktionstemperatur im Bereich von 80 bis 140 °C, besonders bevorzugt 80 bis 130 °C und ganz besonders bevorzugt 80 bis 120 °C ausgeführt. Die benötigte Reaktionszeit für die katalytische Reaktion beträgt vorzugsweise 30 Minuten bis 5 Stunden, besonders bevorzugt 0,75 Stunden bis 4,5 Stunden und ganz besonders bevorzugt 0,75 bis 3,0 Stunden.

Diese Reaktionszeiten sind im Vergleich zur EP 0 043 933 erheblich niedriger. Aus den Beispielen der Druckschrift geht hervor, dass die Reaktionszeiten hier bei bis zu 20 Stunden liegen und daher etwa zwanzig Mal höher sind als bei dem erfindungsgemäßen Verfahren. Die Reaktionstemperatur ist ähnlich hoch und dies bedeutet einen etwa zwanzigfach höheren Energieaufwand, um die Reaktion durchzuführen.

Das erfindungsgemäße Verfahren besitzt daher den Vorteil, dass es kostengünstiger und wirtschaftlicher durchgeführt werden kann und auch aufgrund der kürzeren Reaktionsdauer einen größeren Umsatz pro Zeiteinheit bietet.

Auch die Ausbeuten des erfindungsgemäßen Verfahrens sind höher als im Stand der Technik. So liegen die Ausbeuten gemäß EP 0 043 933 in den Beispielen im Bereich von 60 bis 78 Gew.-%. Im Vergleich dazu werden bei dem erfindungsgemäßen Verfahren Gesamtausbeuten von 80 bis 95 Gew.-%, wie in den Beispielen definiert, erhalten.

In bevorzugter Weise werden die Ausgangssubstanzen aromatisches Amin und Aldehyd in einem Mol-Verhältnis von 5 : 1 bis 15 : 1, vorzugsweise 7 : 1 bis 12 : 1 und ganz besonders bevorzugt 10 : 1 eingesetzt. Es ist bevorzugt, dass das Amin im Überschuss eingesetzt wird, da hierdurch die Selektivität erhöht wird. Überschüssiges Amin kann nach Abschluss der Reaktion abdestilliert werden.

Das erfindungsgemäße Verfahren kann in bevorzugter Weise kontinuierlich, diskontinuierlich oder semikontinuierlich durchgeführt werden.

In bevorzugter Ausführung wird die Reaktionsdurchführung in einem Rührkessel, einer Rührkesselkaskade, einem Strömungsrohr, einem oder mehreren Festbettreaktoren oder einer Kolonne durchgeführt. Die katalytische Reaktion wird an einem heterogenen Katalysator durchgeführt.

Für die Durchführung des erfindungsgemäßen Verfahrens werden die Ausgangssubstanzen aromatisches Amin und Aldehyd kontinuierlich oder diskontinuierlich gemischt. Es erfolgt dann die katalytische Reaktion bei Temperaturen im Bereich von 80 bis 140 °C. Anschließend wird das Isomerengemisch der Diaminodiphenylalkane nach üblichen Trennmethoden isoliert.

Die nachfolgenden Beispiele sollen die Erfindung näher erläutern.

**Beispiele**

1. 93,1 g Anilin und 10 g des feuchten, sulfongruppenhaltigen Ionenaustauscherharzes Amberlyst 35Wet, wurden in einem Rührbehälter unter $N_2$ Atmosphäre zusammengegeben und unter Rühren auf 120 °C erwärmt. Bei dieser Temperatur wurden 8,1 g Formaldehydlösung (37 Gew.-% Formaldehyd in Wasser), entsprechend einem Molverhältnis von Anilin / Formalin von 10:1, innerhalb von 60 Minuten zudosiert. Das eingesetzte Ionenaustauscherharz weist laut Produktdatenblatt des Herstellers eine Konzentration der aciden Zentren von mindestens 5,2 eq/kg und einen durchschnittlichen Porendurchmesser von 300 Å (entspricht 30 nm) auf.

Die Zusammensetzung des Reaktionsproduktes (Gaschromatographie-Analyse nach Abzug des im Überschuss vorliegenden Anilins) stellt sich wie folgt dar: 0,9 Gew.-% 2,2 MDA; 16,8 Gew.-% 2,4 MDA; 70,6 Gew.-% 4,4 MDA; 0,06 Gew.-% N-Methyl- MDA und 11,7 Gew.-% Mehrkernverbindungen. Die Ausbeute betrug 88,2 Gew.-%.

2. 93,1 g Anilin und 10 g des feuchten, sulfongruppenhaltigen Ionenaustauscherharzes Amberlyst 39Wet, wurden in einem Rührbehälter unter $N_2$ Atmosphäre zusammengegeben und unter Rühren auf 120 °C erwärmt. Bei dieser Temperatur wurden 8,1 g Formaldehydlösung (37 Gew.-% Formaldehyd in Wasser), entsprechend einem Molverhältnis von Anilin / Formalin von 10 : 1, innerhalb von 60 Minuten zudosiert. Das eingesetzte Ionenaustauscherharz weist laut Produktdatenblatt des Herstellers eine Konzentration der aciden Zentren von mindestens 5,0 eq/kg und einen durchschnittlichen Porendurchmesser von 230 Å (entspricht 23 nm) auf.

Die Zusammensetzung des Reaktionsproduktes (Gaschromatographie-Analyse nach Abzug des im Überschuss vorliegenden Anilins) stellt sich wie folgt dar: 1,5 Gew.-% 2,2 MDA; 17,9 Gew.-% 2,4 MDA; 66,8 Gew.-% 4,4 MDA; 0,06 Gew.-% N-Methyl- MDA und 13,8 Gew.-% Mehrkernverbindungen. Die Ausbeute betrug 86,1 Gew.-%.

3. 93,1 g Anilin und 10 g des feuchten, sulfongruppenhaltigen Ionenaustauscherharzes Amberlyst 70Wet wurden in einem Rührbehälter unter $N_2$ Atmosphäre zusammengegeben und unter Rühren auf 120 °C erwärmt. Bei dieser Temperatur wurden 8,1 g Formaldehydlösung (37 Gew.-% Formaldehyd in Wasser), entsprechend einem Molverhältnis von Anilin / Formalin von 10 : 1, innerhalb von 60 Minuten zudosiert. Das eingesetzte Ionenaustauscherharz weist laut Produktdatenblatt des Herstellers eine Konzentration der aciden Zentren von mindestens 2,55 eq/kg und einen durchschnittlichen Porendurchmesser von 220 Å (entspricht 22 nm) auf.

Die Zusammensetzung des Reaktionsproduktes (Gaschromatographie-Analyse nach Abzug des im Überschuss vorliegenden Anilins) stellt sich wie folgt dar: 1,1 Gew.-% 2,2 MDA; 15,9 Gew.-% 2,4 MDA; 69,4 Gew.-% 4,4 MDA; 0,03 Gew.-% N-Methyl- MDA und 13,6 Gew.-% Mehrkernverbindungen. Die Ausbeute betrug 86,3 Gew.-%.

Die Beispiele zeigen, dass das geforderte Isomerenverhältnis ohne weitere Aufreinigungs- oder Destillationsschritte erzeugt werden kann. Die Selektivität (S) der Reaktion errechnet sich aus dem Verhältnis zwischen der gebildeten Stoffmenge des gewünschten Produktes (P) (hier die Summe der 2,2', 2,4'und 4,4'-Isomere des MDA) und der umgesetzten Stoffmenge der Schlüsselkomponente (K) (hier Formaldehyd) unter Berücksichtigung der stöchiometrischen Zahlen (v). Für den Satzbetrieb gilt daher:

$$S_P = \frac{n^0}{n_K^0 - n_K} \cdot \frac{|v_K|}{v_P} \cdot 100$$

Nach den angegebenen Minuten wurde der vollständige Umsatz des eingesetzten Formaldehyds erreicht. Daher ist die Selektivität der Reaktion mit der Ausbeute identisch.

**Patentansprüche**

1. Verfahren zur Herstellung von Diaminodiphenylalkanen wobei ein aromatisches Amin, das substituiert oder unsubstituiert sein kann, mit einem $C_1$-$C_3$ Aldehyd in Gegenwart eines heterogenen Katalysators umgesetzt wird, wobei der Katalysator ein mesoporöser saurer Ionenaustauscher auf Basis eines Divinylbenzol/Styrol-Copolymerisates ist und der Katalysator acide Zentren gemessen nach DIN 54 403 in einer Konzentration von 2 bis 6 eq/kg aufweist und der durchschnittliche Porendurchmesser der Katalysatorteilchen gemessen nach ASTM D 4222 22 bis 30 nm beträgt, und wobei der Gehalt an Mehrkernverbindungen mit mehr als zwei aromatischen Kernen im gebildeten Reaktionsgemisch > 10 und ≤ 15 Gew.-% beträgt, und wobei die Reaktionstemperatur der katalytischen Reaktion im Bereich von 80 bis 140°C liegt, mit der Maßgabe, dass der Katalysator ausgewählt wird aus der Gruppe bestehend aus Amberlyst$^R$ 35Wet, Amberlyst$^R$ 39Wet und Amberlyst$^R$ 70Wet.

2. Verfahren nach Anspruch 1, **dadurch gekennzeichnet, dass** als Aldehyd Formaldehyd eingesetzt wird.

3. Verfahren nach Anspruch 1 oder 2, **dadurch gekennzeichnet, dass** als Amin Anilin eingesetzt wird.

4. Verfahren nach einem oder mehreren der Ansprüche 1 bis 3, **dadurch gekennzeichnet, dass** Diaminodiphenylmethan hergestellt wird.

5. Verfahren nach einem oder mehreren der Ansprüche 1 bis 4, **dadurch gekennzeichnet, dass** das Isomerenverhältnis des erhaltenen Diaminodiphenylalkans wie folgt verteilt ist:

| | |
|---|---|
| 64 bis 85 Gew.-% | 4,4' Diaminodiphenylalkan |
| 3 bis 20 Gew.-% | 2,4' Diaminodiphenylalkan |
| ≥ 1,0 Gew.-% bis ≤ 2 Gew.-% | 2,2'Diaminodiphenylalkan |

6. Verfahren nach Anspruch 5, **dadurch gekennzeichnet, dass** das Isomerenverhältnis des erhaltenen Diaminodiphenylmethans wie folgt verteilt ist:

| | |
|---|---|
| 64 bis 85 Gew.-% | 4,4' Diaminodiphenylmethan |
| 3 bis 20 Gew.-% | 2,4' Diaminodiphenylmethan |
| ≥ 1,0 Gew.-% bis ≤ 2 Gew.-% | 2,2'Diaminodiphenylmethan |

7. Verfahren nach einem oder mehreren Ansprüchen 1 bis 6, **dadurch gekennzeichnet, dass** der Gehalt an N-Methylverbindungen ≤ 1 Gew.-% beträgt.

8. Verfahren nach einem oder mehreren Ansprüchen 1 bis 6, **dadurch gekennzeichnet, dass** der Gehalt an Mehrkernverbindungen < 15 Gew.-% beträgt.

9. Verfahren nach einem oder mehreren der Ansprüche 1 bis 8, **dadurch gekennzeichnet, dass** die Reaktionszeit für die katalytische Reaktion 30 min bis 5 Stunden beträgt.

10. Verfahren nach einem oder mehreren der Ansprüche 1 bis 9, **dadurch gekennzeichnet, dass** das Mol-Verhältnis von Amin zu Aldehyd 5 : 1 bis 15 : 1 beträgt.

11. Verfahren nach einem oder mehreren der Ansprüche 1 bis 10, **dadurch gekennzeichnet, dass** der Katalysator in trockener oder feuchter Form eingesetzt werden kann.

12. Verfahren nach einem oder mehreren der Ansprüche 1 bis 11, **dadurch gekennzeichnet, dass** das Verfahren kontinuierlich, diskontinuierlich oder semikontinuierlich durchgeführt wird.

13. Verfahren nach einem oder mehreren der Ansprüche 1 bis 12, **dadurch gekennzeichnet, dass** die Reaktion in einem Rührkessel, einer Rührkesselkaskade, einem Strömungsrohr, einem Festbettreaktor oder in einer Kolonne durchgeführt wird.

**14.** Verfahren nach einem oder mehreren der Ansprüche 1 bis 13, **dadurch gekennzeichnet, dass** die Reaktionstemperatur der katalytischen Reaktion im Bereich von 80 bis 130 °C und ganz besonders bevorzugt 80 bis 120 °C beträgt.

**Claims**

**1.** Process for preparing diaminodiphenylalkanes, where an aromatic amine, which may be substituted or unsubstituted, is reacted with a $C_1$-$C_3$ aldehyde in the presence of a heterogeneous catalyst, the catalyst being a mesoporous acidic ion exchanger based on a divinylbenzene/styrene copolymer, and the catalyst having acidic centres, measured in accordance with DIN 54 403, in a concentration of 2 to 6 eq/kg, and the average pore diameter of the catalyst particles, measured in accordance with ASTM D 4222, being 22 to 30 nm, and the polynuclear compound content, said compounds having more than two aromatic nuclei, of the reaction mixture formed being > 10% and ≤ 15% by weight, and where the reaction temperature of the catalytic reaction is situated in the range from 80 to 140°C, with the proviso that the catalyst is selected from the group consisting of Amberlyst[R] 35Wet, Amberlyst[R] 39 Wet and Amberlyst[R] 70 Wet.

**2.** Process according to Claim 1, **characterized in that** formaldehyde is used as aldehyde.

**3.** Process according to Claim 1 or 2, **characterized in that** aniline is used as amine.

**4.** Process according to one or more of Claims 1 to 3, **characterized in that** diaminodiphenylmethane is prepared.

**5.** Process according to one or more of Claims 1 to 4, **characterized in that** the isomer ratio of the diaminodiphenylalkane obtained is distributed as follows:

64% to 85% by weight
4,4'-diaminodiphenylalkane
3% to 20% by weight
2,4'-diaminodiphenylalkane
≥ 1.0% to ≤ 2% by weight
2,2'-diaminodiphenylalkane.

**6.** Process according to Claim 5, **characterized in that** the isomer ratio of the diaminodiphenylmethane obtained is distributed as follows:

64% to 85% by weight
4,4'-diaminodiphenylmethane
3% to 20% by weight
2,4'-diaminodiphenylmethane
≥ 1.0% to ≤ 2% by weight
2,2'-diaminodiphenylmethane.

**7.** Process according to one or more of Claims 1 to 6, **characterized in that** the N-methyl compounds content is ≤ 1% by weight.

**8.** Process according to one or more of Claims 1 to 6, **characterized in that** the polynuclear compounds content is < 15% by weight.

**9.** Process according to one or more of Claims 1 to 8, **characterized in that** the reaction time for the catalytic reaction is 30 min to 5 hours.

**10.** Process according to one or more of Claims 1 to 9, **characterized in that** the molar ratio of amine to aldehyde is 5:1 to 15:1.

**11.** Process according to one or more of Claims 1 to 10, **characterized in that** the catalyst can be used in dry or moist form.

**12.** Process according to one or more of Claims 1 to 11, **characterized in that** the process is carried out continuously, batchwise or semi-batchwise.

**13.** Process according to one or more of Claims 1 to 12, **characterized in that** the reaction is carried out in a stirred tank, a stirred-tank cascade, a flow tube, a fixed-bed reactor or in a column.

**14.** Process according to one or more of Claims 1 to 13, **characterized in that** the reaction temperature of the catalytic reaction is in the range from 80 to 130°C and very preferably 80 to 120°C.

**Revendications**

**1.** Procédé de fabrication de diaminodiphénylalcanes, selon lequel une amine aromatique, qui peut être substituée ou non substituée, est mise en réaction avec un aldéhyde en $C_1$-$C_3$ en présence d'un catalyseur hétérogène, le catalyseur étant un échangeur d'ions acide mésoporeux à base d'un copolymère de divinylbenzène/styrène et le catalyseur comprenant des centres acides mesurés selon DIN 54 403 en une concentration de 2 à 6 éq./kg et le diamètre de pores moyen des particules de catalyseur, mesuré selon ASTM D 4222, étant de 22 à 30 nm, et la teneur en composés polynucléaires contenant plus de deux noyaux aromatiques dans le mélange réactionnel formé étant > 10 et ≤ 15 % en poids, et la température de réaction de la réaction catalytique se situant dans la plage allant de 80 à 140 °C, à condition que le catalyseur soit choisi dans le groupe constitué par Amberlyst$^R$ 35Wet, Amberlyst$^R$ 39Wet et Amberlyst$^R$ 70Wet.

**2.** Procédé selon la revendication 1, **caractérisé en ce que** le formaldéhyde est utilisé en tant qu'aldéhyde.

**3.** Procédé selon la revendication 1 ou 2, **caractérisé en ce que** l'aniline est utilisée en tant qu'amine.

**4.** Procédé selon une ou plusieurs des revendications 1 à 3, **caractérisé en ce que** du diaminodiphénylméthane est fabriqué.

**5.** Procédé selon une ou plusieurs des revendications 1 à 4, **caractérisé en ce que** le rapport entre les isomères du diaminodiphénylalcane obtenu présente la répartition suivante :

| | |
|---|---|
| 64 à 85 % en poids | 4,4'-diaminodiphénylalcane |
| 3 à 20 % en poids | 2,4'-diaminodiphénylalcane |
| ≥ 1,0 % en poids à ≤ 2 % | diaminodiphénylalcane. en poids 2,2'- |

**6.** Procédé selon la revendication 5, **caractérisé en ce que** le rapport entre les isomères du diaminodiphénylméthane obtenu présente la répartition suivante :

| | |
|---|---|
| 64 à 85 % en poids | 4,4'-diaminodiphénylméthane |
| 3 à 20 % en poids | 2,4'-diaminodiphénylméthane |
| ≥ 1,0 % en poids à ≤ 2 % | en poids 2,2'-diaminodiphénylméthane. |

**7.** Procédé selon une ou plusieurs des revendications 1 à 6, **caractérisé en ce que** la teneur en composés N-méthyle est ≤ 1 % en poids.

**8.** Procédé selon une ou plusieurs des revendications 1 à 6, **caractérisé en ce que** la teneur en composés polynucléaires est < 15 % en poids.

**9.** Procédé selon une ou plusieurs des revendications 1 à 8, **caractérisé en ce que** la durée de réaction pour la réaction catalytique est de 30 minutes à 5 heures.

**10.** Procédé selon une ou plusieurs des revendications 1 à 9, **caractérisé en ce que** le rapport molaire entre l'amine et l'aldéhyde est de 5:1 à 15:1.

**11.** Procédé selon une ou plusieurs des revendications 1 à 10, **caractérisé en ce que** le catalyseur peut être utilisé sous forme sèche ou humide.

12. Procédé selon une ou plusieurs des revendications 1 à 11, **caractérisé en ce que** le procédé est réalisé de manière continue, discontinue ou semi-continue.

13. Procédé selon une ou plusieurs des revendications 1 à 12, **caractérisé en ce que** la réaction est réalisée dans une cuve agitée, une cascade de cuves agitées, un tube d'écoulement, un réacteur à lit fixe ou dans une colonne.

14. Procédé selon une ou plusieurs des revendications 1 à 13, **caractérisé en ce que** la température de réaction de la réaction catalytique se situe dans la plage allant de 80 à 130 °C et de manière tout particulièrement préférée de 80 à 120 °C.

## IN DER BESCHREIBUNG AUFGEFÜHRTE DOKUMENTE

**In der Beschreibung aufgeführte Patentdokumente**

- US 4294981 A
- DE 1230033 A
- DE 1493431 A
- US 4071558 A
- US 4039580 A
- US 4039581 A
- EP 0043933 A1
- EP 0043933 A